Europäisches Patentamt

European Patent Office

Office européen des brevets

(11) Veröffentlichungsnummer: **0 405 485 B1**

# (12) EUROPÄISCHE PATENTSCHRIFT

(45) Veröffentlichungstag der Patentschrift: **28.09.94**

(21) Anmeldenummer: **90112225.9**

(22) Anmeldetag: **27.06.90**

(51) Int. Cl.⁵: **C07C 45/38**, C07C 45/39, C07C 47/04

(54) Verfahren zur Herstellung von Carbonylverbindungen.

(30) Priorität: **30.06.89 DE 3921452**

(43) Veröffentlichungstag der Anmeldung:
**02.01.91 Patentblatt 91/01**

(45) Bekanntmachung des Hinweises auf die
Patenterteilung:
**28.09.94 Patentblatt 94/39**

(84) Benannte Vertragsstaaten:
**DE GB NL**

(56) Entgegenhaltungen:
US-A- 3 529 020
US-A- 3 692 840
US-A- 4 359 587

CHEMICAL ABSTRACTS, Band 88, 1978, Seite 493, Spalte 1, Zusammenfassung Nr.50224p, Columbus, Ohio, US; G.O. CHIVADZE et al.: "Oxidative dehydrogenation ofmethanol on AgNaX zeolite"

CHEMICAL ABSTRACTS, Band 104, Nr. 15, April 1986, Seite 661, Spalte 2,Zusammenfassung Nr. 129457z, Columbus, Ohio, US; T.G. ALKHAZOV et al.:"Oxidation of isoamyl alcohol on silver catalysts"

(73) Patentinhaber: **HOECHST AKTIENGESELL-SCHAFT**

**D-65926 Frankfurt (DE)**

(72) Erfinder: **Beck, Horst-Philipp, Prof.-Dr.**
**Scheidter Strasse 158**
**D-6600 Saarbrücken (DE)**
Erfinder: **Emig, Gerhard, Prof.-Dr.**
**Vogelherd 157**
**D-8520 Erlangen (DE)**
Erfinder: **Wiesgickl, Günther, Dr.**
**Schillerring 23**
**D-8751 Grosswallstadt (DE)**
Erfinder: **Burg, Karlheinz, Dr.**
**Eichenweg 18**
**D-6200 Wiesbaden (DE)**
Erfinder: **Mück, Karl-Friedrich, Dr.**
**Schnitterweg 7**
**D-6200 Wiesbaden (DE)**

**Beschreibung**

Die Erfindung bezieht sich auf ein Verfahren zur Herstellung von Carbonylverbindungen durch Oxydehydrierung von $(C_1-C_4)$-Alkoholen bei einer Temperatur von 400 bis 700° C in Gegenwart eines Silikatkatalysators, der mit Silber und Silberionen dotiert ist.

Gerüstsilikatkatalysatoren, z.B. Zeolithe, werden großtechnisch bereits für die nichtoxidative Umwandlung von Kohlenwasserstoffen als Katalysatoren eingesetzt, z. B. beim katalytischen Cracken.

Es ist auch bekannt, daß organische Stoffe mit Hilfe von Zeolithkatalysatoren selektiv oxidiert werden können, wie dies z.B. bei der Acetonherstellung aus Propen der Fall ist. Hier wird bei einem Umsatz von 50 % eine Selektivität von 90 % erzielt. Aussagen über die Langzeitaktivität und das Alterungsverhalten der verwendeten Katalysatoren werden dabei nicht gemacht.

Weiterhin wird angenommen, daß Zeolithe infolge ihrer besonderen Struktur zur Beschleunigung der Totaloxidation von organischen Stoffen zu $CO_2$ und Wasser neigen.

Die Herstellung von bestimmten Katalysatoren auf Zeolith-Basis wird in der DDR-Patentschrift 113 173 beschrieben. Hierbei erfolgt der Einbau bestimmter Metalle wie Vanadium und/oder Titan in die Zeolithe durch Ionenaustausch.

Für die industriell besonders bedeutsamen Oxidehydrierungen von $C_1$-$C_4$-Alkoholen, z.B. von Methanol zu Formaldehyd wird häufig ein Katalysator "Silber auf Bimstein" bzw. "Silber auf $Al_2O_3$" verwendet. In der deutschen Patentschrift 30 37 536 wird auf die Herstellung eines Silberkatalysators eingegangen, der durch Tränken des Trägers, welcher aus $Al_2O_3$ und $SiO_2$ in Form von Cristobalit besteht, mit Silbernitratlösung synthetisiert wird.

Für die Herstellung von Formaldehyd aus Methanol wird auch ein Katalaysator beschrieben, bestehend aus einem Träger aus Metall oder Keramik, der mit metallischem Kupfer, Silber, Gold oder Eisen beschichtet oder imprägniert ist (DE-OS 28 16 471).

Die genannten Katalysatoren, die bei Verfahren zur Herstellung von Carbonylverbindungen durch oxidative Dehydrierung von $C_1$-$C_4$-Alkoholen eingesetzt werden, weisen jedoch nur relativ niedrige Ausbeuten an Endprodukt, bezogen auf den durch- und umgesetzten Rohstoff, auf, weiterhin ist der Grad der Umwandlung des Rohstoffs und die Qualität der Produkte meist noch unbefriedigend. Als unerwünschtes Nebenprodukt wird bei den bekannten Verfahren häufig auch Ameisensäure gebildet. Es bestand daher die Aufgabe, das Verfahren zur Herstellung von Oxoverbindungen weiter zu verbessern.

Das Verfahren der Oxydehydrierung von $(C_1-C_4)$-Alkoholenfindet beispielsweise Verwendung bei der Herstellung von Formaldehyd aus Methanol, von Aceton aus Isopropanol und von Methylethylketon aus dem sekundären Butanol.

Formaldehyd ist ein wichtiger Rohstoff für die Industrie. Er wird beispielsweise als Desinfektions- und Desodorierungsmittel verwendet. Weiterhin ist er Ausgangsprodukt für zahlreiche Synthesen in der chemischen Industrie und wird überwiegend zu der Herstellung von Kunststoffen und pharmazeutischen Präparaten verwendet. Darüberhinaus findet er auch in der Farbstoff- und Hilfsmittelindustrie Verwendung.

Die Erfindung betrifft ein Verfahren zur Herstellung von Carbonylverbindungen durch Umsetzung von $(C_1-C_4)$-Alkoholen in Gegenwart von Sauerstoff unter Verwendung eines Katalysators bei erhöhter Temperatur, bei dem die Umsetzung bei einer Temperatur von 400 bis 700, vorzugsweise 500 bis 600 °C in Gegenwart eines Katalysators, der aus einem Silberhalogenid und elementares Silber enthaltenden Silikat-Komplex besteht, durchgeführt wird. Der Katalysator kann durch die Formel

$$M_{2/n}O \cdot v\ Ag^0 \cdot w\ AgX \cdot Al_2O_3 \cdot x\ SiO_2 \cdot y\ H_2O \qquad (1)$$

gekennzeichnet werden, bei dem M ein Metallatom der Wertigkeit n, $Ag^0$ elementares Silber, X ein Halogenatom, vorzugsweise Chlor oder Brom und v,w,x und y stöchiometrische Koeffizienten bedeuten. Vorzugsweise besitzt der Katalysator die Formel

$$Na_2O \cdot 1{,}4\ Ag \cdot 0{,}4\ AgCl \cdot Al_2O_3 \cdot 2{,}47\ SiO_2 \cdot 3{,}9\ H_2O \qquad (2).$$

Die Herstellung der eingesetzten Silikatkatalysatoren ist in der deutschen Patentanmeldung P 39 21 450.8, Titel: "Katalysator für selektive Oxidationsreaktionen" beschrieben, die am selben Tag eingereicht worden ist und auf die hiermit Bezug genommen wird. Der Katalysator besitzt eine Teilchengröße von 0,5 bis 5 mm, vorzugsweise 0,5 bis 2 mm.

Das erfindungsgemäße Verfahren wird derart durchgeführt, daß die gasförmigen Ausgangskomponenten gemischt und vorgewärmt und anschließend dem Verdampfer zugeführt werden. Gleichzeitig wird auch die Alkoholkomponente in den Verdampfer geleitet. Das dabei entstehende Gasgemisch wird dann durch den

2

Reaktor geleitet, in dem sich der auf eine Temperatur von 400 bis 700 °C, vorzugsweise 500 bis 600 °C erhitzte Katalysator befindet. Die Reaktionstemperaturen liegen gegenüber denen bei dem Verfahren bei Einsatz von Katalysatoren aus reinem Silber deutlich niedriger.

Als Material für die Ausgestaltung des Reaktors kommen die üblichen bisher bekannten Materialien in Frage. Es hat sich jedoch die Verwendung von Quarzglas als besonders vorteilhaft herausgestellt.

Die Anordnung des Katalysators in der Apparatur kann nach bekannten Methoden erfolgen. Vorteilhaft ist es, mit einem Festbettreaktor zu arbeiten, bei dem sichergestellt ist, daß der Austrag von sehr feinen Katalysatorpartikeln durch den Gasstrom auf geeignete Weise vermieden wird.

Zur Umsetzung in Carbonylverbindungen kommen bei dem erfindungsgemäßen Verfahren aliphatische Alkohole mit 1 bis 4 C-Atomen in der Alkylgruppe in Frage, beispielsweise Methanol, Isopropanol und sekundärer Butylalkohol. Die oxidative Dehydrierung erfolgt in Gegenwart von Sauerstoff. Hierunter ist auch zu verstehen, daß als oxidierendes Medium Luft eingesetzt wird. Wird reiner Sauerstoff eingesetzt, ist die zusätzliche Verwendung eines inerten Gases, vorzugsweise Stickstoff, zweckmäßig.

Durch das erfindungsgemäße Verfahren wird der Umsatz von $C_1$-$C_4$-Alkoholen überraschenderweise erhöht und liefert Carbonylverbindungen mit verbesserter Selektivität und Ausbeute.

Die in den Beispielen angegebenen Meßgrößen wurden wie folgt berechnet:

$$\text{Umsatz (in \%)} = \frac{\text{umgesetzes Methanol (mol)}}{\text{zudosiertes Methanol (mol)}} \cdot 100$$

$$\text{Ausbeute (in \%)} = \frac{\text{gebildeter Formaldehyd (mol)}}{\text{zudosiertes Methanol (mol)}} \cdot 100$$

$$\text{Selektivität (in \%)} = \frac{\text{gebildeter Formaldehyd (mol)}}{\text{umgesetztes Methanol (mol)}} \cdot 100$$

Mit dem bei relativ niedrigen Temperaturen durchgeführten Verfahren werden z.B. hohe Ausbeuten erzielt, der Anteil an Kohlenmonoxid ist gering und das Produktgas enthält außer Kohlendioxid keine weiteren bestimmbaren Nebenprodukte bei geringem Wasser/Formaldehyd-Verhältnis.

**Beispiele**

Herstellung der Katalysatoren

1) 10,71 g Zeolith 13X (Formel: $Na_2O \cdot Al_2O_3 \cdot 2,47\ SiO_2 \cdot 3,9\ H_2O$) wurden in ein Becherglas gegeben, mit 500 ml 0,1 n $AgNO_3$-Lösung versetzt und 4 Stunden unter Lichtausschluß kräftig gerührt. Nach Filtration wurde der Rückstand mit 50 ml Wasser gewaschen und erneut mit 400 ml 0,1 n Silbernitratlösung versetzt, 4 Stunden unter Lichtausschluß gerührt, anschließend abfiltriert und die erhaltene feste Substanz an der Luft getrocknet. 7,88 g dieser Substanz wurden bei 350 °C unter vermindertem Druck entwässert und dann mit Wasserstoff bei einem Druck von 0,6 bar für 20 Minuten bei der gleichen Temperatur reduktiv behandelt. Anschließend wurde der Wasserstoff entfernt und der Rückstand auf 270 °C abgekühlt. Die Festsubstanz wurde anschließend in Sauerstoff (0,67 bar) für 8 Minuten bei dieser Temperatur behandelt und nach Entfernung des Sauerstoffs auf ca. 25 °C abgekühlt. 3,5 g der erhaltenen Substanz wurden in 200 ml Wasser aufgeschlämmt und unter Rühren 5 ml konzentrierte Salzsäure zugegeben. Fünf Minuten später erfolgte die Zudosierung von 25 ml halbkonzentrierter Ammoniaklösung (7 mol/l) und 17 g Natriumnitrat. Diese Suspension wurde 1 Stunde gerührt, anschließend filtriert und der Rückstand mit insgesamt 200 ml Wasser gewaschen. Die erhaltene Substanz wurde an der Luft getrocknet und zu Pellets mit 6 mm Durchmesser gepresst, die anschließend zerkleinert wurden.

2) 8,51 g Zeolith 13X (Formel: $Na_2O \cdot Al_2O_3 \cdot 2,47\ SiO_2 \cdot 3,9\ H_2O$) wurden in ein Becherglas gegeben, mit 400 ml 0,1 n $AgNO_3$-Lösung versetzt und 3 Stunden unter Lichtausschluß kräftig gerührt. Nach Filtration wurde der Rückstand mit 50 ml Wasser gewaschen und erneut mit 400 ml 0,1 n Silbernitratlösung versetzt, 3 Stunden unter Lichtausschluß gerührt, anschließend filtriert und die erhaltene Festsubstanz an der Luft getrocknet. 6,35 g dieser Substanz wurden bei 330 °C unter vermindertem Druck entwässert und dann mit reinem Wasserstoff bei einem Druck von 0,67 bar für 25 Minuten reduziert. Anschließend wurde die Substanz unter vermindertem Druck auf 170 °C abgekühlt, mit Chlor bei einem Druck von 0,6 bar für 30 Minuten oxidiert, und unter vermindertem Druck auf Raumtemperatur abgekühlt. 4 g der so erhaltenen Substanz wurden in 200 ml Wasser aufgeschlämmt und 20 g Natriumnitrat sowie 30 ml halbkonzentrierte Ammoniaklösung zudosiert. Diese Suspension wurde 20 Minuten gerührt, dann filtriert und der Rückstand mit insgesamt 250 ml Wasser gewaschen. Die erhaltene Substanz wurde an der Luft getrocknet und zu Pellets mit 6 mm Durchmesser gepresst, die anschließend zerkleinert wurden.

3) (Vergleich 1) 6,7 g Zeolith 13X (Formel: $Na_2O \cdot Al_2O_3 \cdot 2,47 \; SiO_2 \cdot 3,9 \; H_2O$) wurden in ein Becherglas gegeben, mit 300 ml 0,1 n $AgNO_3$-Lösung versetzt und 4 Stunden unter Lichtausschluß kräftig gerührt. Nach Filtration wurde der Rückstand mit 50 ml Wasser gewaschen und erneut mit 300 ml 0,1 n Silbernitratlösung versetzt, 4 Stunden unter Lichtausschluß gemischt, anschließend abfiltriert und die erhaltene feste Substanz an der Luft getrocknet. 7,5 g dieser Substanz wurden bei 350 °C unter vermindertem Druck entwässert und dann mit Wasserstoff bei einem Druck von 0,6 bar für 20 Minuten bei der gleichen Temperatur reduktiv behandelt.

Nach Verminderung des Druckes wurde auf Raumtemperatur abgekühlt, die erhaltene Substanz in 200 ml Wasser aufgeschlämmt und mit 25 ml halbkonzentrierter Ammoniaklösung (7 mol/l) und 15 g Natriumnitrat versetzt.

Diese Suspension wurde gerührt, anschließend filtriert und der Rückstand mit insgesamt 100 ml Wasser gewaschen. Die erhaltene Substanz wurde an der Luft getrocknet und zu Pellets mit 6 mm Durchmesser gepresst, die anschließend zerkleinert wurden.

4) (Vergleich 2) Der Vergleichskatalysator 2 besteht aus annähernd kugelförmigem, elektrolytisch abgeschiedenem Silber mit einem Partikeldurchmesser von 1 ± 0,5 mm.

5) Von den Katalysatoren der Beispiele 1 bis 4 wurde jeweils eine Korngrößenfraktion von 1 mm ± 0,5 für die Umwandlung von Methanol in Formaldehyd gemäß dem erfindungsgemäßen Verfahren ausgesucht. Die Katalysatoren wurden jeweils in ein Quarzrohr mit einem Innendurchmesser von 12 mm gegeben, das auf die angeführten Reaktionstemperaturen aufgeheizt wurde, wobei in den Beispielen 1 und 4 die Schütthöhe des Katalysators gleich war. Die gasförmigen Ausgangsstoffe wurden in den in der Tabelle angegebenen Mengen dosiert, gemischt und vorgewärmt, und anschließend einem vor dem Reaktionsteil vorhandenen Verdampfer zugeführt. Gleichzeitig wurden die jeweils eingesetzten Methanolmengen ebenfalls in den Verdampfer eingeleitet. Das dabei entstehende Gasgemisch wurde durch das Reaktionsrohr geleitet.

Das Produktgas wurde gaschromatographisch analysiert. Die entsprechenden Daten und Ergebnisse sind in der nachfolgenden Tabelle zusammengefaßt, wobei der Sauerstoffumsatz stets bei 100 % lag.

In der Tabelle bedeutet:

Kat.: Katalysator

T: Reaktionstemperatur

$O_2$: Sauerstoff

Ausb.: Ausbeute an Fa

Fa: Formaldehyd

Einw.: Einwaage an Katalysator

Me: Methanol

$N_2$: Stickstoff

Ums.: Umsatz an Methanol

Sel.: Selektivität zu Fa

$H_2O$/Fa: Wasser/Formaldehyd-Verhältnis im Produktgas

**Tabelle**

| Kat. (Beisp.) | Kat. Einw. (g) | T (°C) | Mol (Me/h) (mol/h) | Mol (N$_2$/h) (mol/h) | Mol (O$_2$/h) (mol/h) | Ums. (%) | Ausb. (%) | Sel. (%) | H$_2$O/Fa (mol/mol) |
|---|---|---|---|---|---|---|---|---|---|
| 1 | 1,00 | 550 | 0,379 | 3,467 | 0,0381 | 33,5 | 32,5 | 97,0 | 0,645 |
| " | " | " | " | 3,437 | 0,0665 | 51,9 | 50,4 | 97,1 | 0,658 |
| " | " | " | " | 3,414 | 0,0871 | 64,3 | 61,4 | 95,4 | 0,735 |
| " | " | " | 0,380 | 3,384 | 0,117 | 76,3 | 69,6 | 91,3 | 0,790 |
| " | " | 600 | 0,358 | 3,265 | 0,0358 | 32,0 | 31,9 | 99,6 | 0,679 |
| " | " | " | " | 3,241 | 0,0627 | 52,2 | 51,1 | 97,9 | 0,667 |
| " | " | " | " | 3,218 | 0,0835 | 64,5 | 62,4 | 96,7 | 0,725 |
| " | " | " | " | 3,194 | 0,1105 | 76,8 | 72,7 | 94,7 | 0,792 |
| 4 (V2) | 5,66 | 550 | 0,379 | 3,467 | 0,0381 | 25,4 | 24,8 | 97,6 | 0,858 |
| " | " | " | " | 3,437 | 0,0665 | 40,8 | 38,8 | 95,1 | 0,863 |
| " | " | " | 0,380 | 3,414 | 0,0871 | 50,7 | 47,3 | 93,2 | 0,898 |
| " | " | " | " | 3,384 | 0,117 | 62,0 | 55,0 | 88,7 | 0,931 |
| " | " | 600 | 0,358 | 3,265 | 0,0358 | 29,3 | 29,1 | 99,3 | 0,759 |
| " | " | " | " | 3,421 | 0,0627 | 47,2 | 45,9 | 97,2 | 0,760 |
| " | " | " | " | 3,218 | 0,0835 | 60,1 | 55,3 | 91,9 | 0,789 |
| " | " | " | " | 3,194 | 0,1105 | 71,9 | 63,0 | 87,7 | 0,859 |
| 2 | 1,65 | 550 | 0,397 | 3,496 | 0,0795 | 55,5 | 53,5 | 96,4 | 0,677 |
| " | " | 600 | " | 3,414 | 0,159 | 85,1 | 76,4 | 89,8 | 0,782 |
| 3 (V1) | 1,52 | 550 | 0,397 | 3,496 | 0,0795 | 55,1 | 52,7 | 95,8 | 0,686 |
| " | " | 600 | " | 3,414 | 0,159 | 87,6 | 75,6 | 86,3 | 0,807 |

Aus der Tabelle ist ersichtlich, daß bei Einsatz der komplexen Silikat-Katalysatoren gemäß dem erfindungsgemäßen Verfahren der Umsatz von Methanol und die Ausbeute an Formaldehyd und Selektivität zu Formaldehyd gegenüber den als Vergleich eingesetzten Katalysatoren steigt. Weiterhin ist das Wasser/Formaldehyd-Verhältnis im Produktgas eindeutig zugunsten der bei dem erfindungsgemäßen Verfahren eingesetzten silberdotierten Silikat-Katalysatoren verschoben. Besonders auffällig ist auch die erhöhte Aktivität dieser Katalysatoren gegenüber einem kugelförmigen Silberkatalysator gemäß Beispiel 4 (Vergleich 2).

**Patentansprüche**

1. Verfahren zur Herstellung von Carbonylverbindungen durch Umsetzung von $(C_1-C_4)$-Alkoholen in Gegenwart von Sauerstoff unter Verwendung eines Katalysators bei erhöhter Temperatur, dadurch gekennzeichnet, daß die Umsetzung bei einer Temperatur von 400 bis 700 °C in Gegenwart eines Katalysators, der aus einem Silberhalogenid und elementares Silber enthaltenden Silikat-Komplex besteht, durchgeführt wird.

2. Verfahren nach Anspruch 1, dadurch gekennzeichnet, daß die Umsetzung bei 500 bis 600 °C durchgeführt wird.

3. Verfahren nach Anspruch 1 oder 2, dadurch gekennzeichnet, daß Methanol umgesetzt wird.

4. Verfahren nach einem oder mehreren der Ansprüche 1 bis 3, dadurch gekennzeichnet, daß als Katalysator ein Gerüstsilikat-Komplex der Formel

$$M_{2/n}O \bullet v\ Ag^0 \bullet w\ AgX \bullet Al_2O_3 \bullet x\ SiO_2 \bullet y\ H_2O \qquad (1)$$

eingesetzt wird, wobei M ein Metallatom der Wertigkeit n, $Ag^0$ ein elementares Silber, X ein Halogenatom und v,w,x und y stöchiometrische Koeffizienten bedeuten.

5. Verfahren nach einem oder mehreren der Ansprüche 1 bis 4, dadurch gekennzeichnet, daß als Katalysator ein Gerüstsilikat-Komplex der Formel

$$Na_2O \bullet 1,4\ Ag \bullet 0,4\ AgCl \bullet Al_2O_3 \bullet 2,47\ SiO_2 \bullet 3,9\ H_2O \qquad (2)$$

eingesetzt wird.

6. Verfahren nach einem oder mehreren der Ansprüche 1 bis 5, dadurch gekennzeichnet, daß die Reaktion in Gegenwart eines inerten Gases, vorzugsweise Stickstoff, durchgeführt wird.

7. Verfahren nach einem oder mehreren der Ansprüche 1 bis 5, dadurch gekennzeichnet, daß ein Alkohol-Luftgemisch zur Reaktion gebracht wird.

8. Verfahren nach einem oder mehreren der Ansprüche 1 bis 7, dadurch gekennzeichnet, daß der Katalysator in Form von annähernd kugelförmigen Partikeln mit Durchmessern im Bereich von 0,5 bis 5 mm, vorzugsweise 0,5 bis 2 mm verwendet wird.

**Claims**

1. A process for the preparation of carbonyl compounds by reacting $(C_1-C_4)$-alcohols in the presence of oxygen using a catalyst at an elevated temperature, which comprises carrying out the reaction at a temperature from 400 to 700 °C in the presence of a catalyst composed of a silicate complex containing silver halide and elementary silver.

2. The process as claimed in claim 1, wherein the reaction is carried out at 500 to 600 °C.

3. The process as claimed in claim 1 or 2, wherein methanol is reacted.

4. The process as claimed in one or more of claims 1 to 3, wherein the catalyst employed is a skeleton silicate complex of the formula

$$M_{2/n}O \bullet v\ Ag^0 \bullet w\ AgX \bullet Al_2O_3 \bullet x\ SiO_2 \bullet y\ H_2O \qquad (1)$$

in which M denotes a metal atom of valence n, $Ag^0$ denotes elementary silver, X denotes a halogen atom and v, w, x and y denote stoichiometric coefficients.

5. The process as claimed in one or more of claims 1 to 4, wherein the catalyst employed is a skeleton silicate complex of the formula

$Na_2O \cdot 1.4\ Ag \cdot 0.4\ AgCl \cdot Al_2O_3 \cdot 2.47\ SiO_2 \cdot 3.9\ H_2O$    (2)

6. The process as claimed in one or more of claims 1 to 5, wherein the reaction is carried out in the presence of an inert gas, preferably nitrogen.

7. The process as claimed in one or more of claims 1 to 5, wherein an alcohol/air mixture is reacted.

8. The process as claimed in one or more of claims 1 to 7, wherein the catalyst is used in the form of approximately spherical particles having diameters within the range from 0.5 to 5 mm, preferably 0.5 to 2 mm.

**Revendications**

1. Procédé de préparation de composés carbonyliques par transformation d'alcools en $C_1$-$C_4$ en présence d'oxygène avec un catalyseur, à de hautes températures, procédé caractérisé en ce que l'on effectue la réaction à une température de 400 à 700 °C en présence d'un catalyseur constitué d'un silicate complexe comprenant un halogénure d'argent et de l'argent élémentaire.

2. Procédé selon la revendication 1, caractérisé en ce que l'on effectue la réaction entre 500 et 600 °C.

3. Procédé selon la revendication 1 ou 2, caractérisé en ce que l'on opère sur du méthanol.

4. Procédé selon une ou plusieurs des revendications 1 à 3, caractérisé en ce que l'on utilise comme catalyseur un complexe à structure de silicate de formule

$M_{2/n}O \cdot v\ Ag^0 \cdot w\ AgX \cdot Al_2O_3 \cdot x\ SiO_2 \cdot y\ H_2O$    (1)

dans laquelle
   M                   désigne un atome de métal de valence n, $Ag^0$ l'argent élémentaire,
   X                   un atome d'halogène et
   v, w, x et y     sont les coefficients stoechiométriques.

5. Procédé selon une ou plusieurs des revendications 1 à 4, caractérisé en ce que l'on utilise comme catalyseur un complexe à structure de silicate de formule

$Na_2O \cdot 1,4\ Ag \cdot 0,4\ AgCl \cdot Al_2O_3 \cdot 2,47\ SiO_2 \cdot 3,9\ H_2O$    (2)

6. Procédé selon une ou plusieurs des revendications 1 à 5, caractérisé en ce que l'on effectue la réaction en présence d'un gaz inerte, de préférence d'azote.

7. Procédé selon une ou plusieurs des revendications 1 à 5, caractérisé en ce que l'on fait réagir un mélange de l'alcool et d'air.

8. Procédé selon une ou plusieurs des revendications 1 à 7, caractérisé en ce que le catalyseur employé est en particules ayant à peu près la forme de billes, de diamètres compris entre 0,5 et 5 mm, de préférence entre 0,5 et 2 mm.